# EUROPEAN PATENT APPLICATION

(11) **EP 4 140 311 A1**
(43) Date of publication of application: **01.03.2023**
(21) Application number: 21193115.9
(22) Date of filing: 25.08.2021
(51) Int. Cl.: A23C 9/127, A61K 35/20, A61K 35/744, A61K 35/747, A61K 36/06

(54) **METHOD TO PRODUCE A NON-KEFIR GRAIN BASED SYNTHETIC KEFIR-LIKE FERMENTED PRODUCT AND METHOD FOR KEFIR-GRAIN FREE CULTIVATION OF KEFIR MICROORGANISM CULTURES**

(71) Applicant: Technische Universität Berlin, 10623 Berlin (DE)
(72) Inventor: Nejati, Fatemeh, 10559 Berlin (DE); Neubauer, Peter, 13467 Berlin (DE); Junne, Stefan, 12163 Berlin (DE)
(74) Representative: Fleuchaus & Gallo Partnerschaft mbB

(57) **Abstract**

The present invention refers to a method to produce a synthetic kefir-like fermentation product by bacterial strain from a raw beverage source.

## Description

In 2019, the Food and Health Survey - conducted by the International Food Information Council - revealed about 23% of consumers seek food and beverages for health benefits. Of these, the majority of consumers sought products for digestive health. Consumers are becoming more aware of the importance of gut microbiota in maintaining immunity and overall health, which is emerging as one of the leading market trends. Probiotic beverages are becoming popular among consumers as instant sources of gut-friendly bacteria. The growing demand for functional and fermented beverages is expected to drive the demand for probiotic beverages in upcoming years. Among the other functional products, according to Fortune Business Insights the global kefir market size was valued at USD 1.23 billion in 2019 and is projected to reach USD 1.84 billion by 2027.

Kefir is a traditional natural fermented product. The conventional/classical product process of kefir is done by adding kefir grains (5-20%) to fresh milk (classically of cow or goat) and allow the milk to be fermented and acidified (reducing the milk pH from 6.5 to below 4.7) in average for 24h. Kefir grains apparently consist of a wide range of microorganisms (which belong to lactic acid bacteria (LAB), yeasts, and acetic acid bacteria) attached to a cauliflower-shaped structure mainly composed of polysaccharides and proteins which serve as a binder to bind the single cells (microorganisms) together.

In the last few years, kefir or the microorganisms that belong to the microbial community of kefir have been a subject of many studies, many health benefits have been attributed to kefir consumption. The health benefits of kefir are various, it is a proven source of different probiotics, prebiotics (mainly kefiran, the exopolysaccharide which is exclusively produced by *Lactobacillus kefiranofaciens* and which play a key role in kefir grain formation and viscosity of grain-based kefir), and various functional compounds (products of microbial metabolic activities). All together these components of kefir make a natural functional food with health promoting effects, including immunomodulatory, anti-cancer, anti-biofilm, antioxidant, anti-viral, cholesterol lowering, anti-toxin, antihypertension and etc. (Rahbar Saadat, et al. 2019; Slattery, et al. 2019). These natural health promoting properties have been used for the development of some commercial health support products.

The exact microbiological composition of kefir grains is still controversial. Various analyses indicate a large variation in the microbial composition of kefir grains. However, a few bacterial species are ubiquitous. In a recent analysis performed in the department of the inventors, it was found out that among six investigated bacteria two Lactobacilli species, i.e. *Lactobacillus kefiranofaciens and Lactobacillus kefiri* (the latter also known as *Lentilactobacillus kefiri)* are detectable in high numbers in all tested milk kefir grains and beverage produced out of these grains, though the other four bacteria species *(Lactococcus (Lc.) lactis, Leuconostoc (Ln.) mesenteroides* and *Acetobacterspp.)* were varying in different milk kefirs (Nejati, et al. 2020).

In addition to bacterial species, various yeasts, which are either lactose fermenting (e.g., *Kluyveromyces (Kl.) marxianus and Dekerra (D.) anomala)* or non-lactose- fermenting (e.g., *Kazachstania (Kz.) unispora, Kz. turicensis and Saccharomyces (S.) cerevisiae)* play important roles in the kefir grain's environment by the very special interactions they undergo with the bacteria.

In case the kefir is produced using kefir grains, the microorganisms contained in the grains (predominantly lactic acid bacteria and yeast) are at least partially also distributed in the milk medium and grow there to receive the kefir. Used kefir grains can be harvested and re-used for producing another kefir.

One obstacle in grain application is that the kefir produced in this classical way is subject and prone to quality fluctuations, as the number and specific microorganism strains and thus the composition of the grains may change during cycles of use and recycle.

Despite of extensive health properties of grain-based kefir consumption, the utilization of the typical natural kefir grains has the drawbacks that the grains are only slowly growing in milk and that the growth of each of the single microorganisms derived from the grains cannot be controlled. Moreover, there is the problem that under unsuitable hygiene conditions, unwanted microorganisms as wild yeast or coliform bacteria may pollute the natural kefir grains which may give rise to toxic and malodorous substances when processed to kefir. Additionally, the end product kefir beverage itself containing at least some of the microorganisms contained in the grains cannot be used as starter culture to inoculate other milk.

These characteristics making the quite complicated and time-consuming traditional method of producing kefir utilizing kefir-grains not feasible at an industrial scale which requires consistently controlled and efficient high growth rates of the microbial components to receive an end product (kefir) of consistently good and reliable quality at high production rate.

Therefore, the industrial production of kefir-like fermented products is performed by using a mixture of defined dairy related microorganisms instead, known as so called kefir "starter cultures". In industrial kefir starter cultures, in order to reduce the time of the non-grain based (i.e., synthetic/artificial) kefir production process, fast growing bacterial strains such as *Lc. lactis ssp., Leuconostoc ssp., Streptococcus thermophilus,* and *Lactobacilli like Lactobacillus plantarum (also known as Lactiplantibacillus plantarum)* a nd *Lactobacillus helveticus* represent the major fractions. However, to the best knowledge of the inventors of the present invention - without being bound by that theory - the two species *L. kefiranofaciens* and *L. kefiri* represent the most important and abundant bacteria of the natural kefir microbial community in classical kefir grain-based beverages. This could be recently shown in, e.g., Wang, *et al.* 2020 or in Nejati *et al.,* 2020. These bacteria are slow growing, but make the structure of kefir grains. Moreover, both Lactobacillus species *(L. kefiranofaciens* and *L. kefiri)* have high ability of aggregation and present otherfunctions that are not still well known due to the very limited studies. Additionally, *L. kefiranofaciens* has a unique role in production of an exopolysaccharide called kefiran, which has critical role in kefir grain formation and possesses many health benefits as recently shown. In general, the average kefiran amount in kefir beverage is approximately around 1.6 to 2.0 g/L depending on the reported source and largely depending how metabolic active *L. kefiranofaciens* is, which in turn depends on its environment it is exposed to, especially if the environment provides a proliferation-supportive milieu for *L. kefiranofaciens.*

Thus, these industrial commercially available kefir grain-based beverages are not essentially representative of the real microbial composition of grain-based kefir beverages. To the best knowledge of the inventors of the present invention none of the commercialized kefir beverages contains cultures of *L. kefiri* alone or of *L. kefiranofaciens* alone or in combination at least not at the levels which are found in natural kefir beverages derived from kefir grains *(L. kefiranofaciens:* a minimum of 1*10⁸cells/mL of classical kefir beverage; *L. kefiri:* a minimum of 1*10⁷cells/mL of classical kefir beverage (cf. Nejati, et al. 2020)).

The health and taste properties of fermented products, which are directly related to the microbial species, namely the organoleptic properties (e.g., taste, smell, touch), metabolite profiles and functional characteristics are prone to a drastic change, if the natural kefir grain cultures are not used as starter cultures. Thus, the synthetic kefirs on the market are detrimentally significant different from an organoleptic point of view compared to classical grain-based produced kefirs.

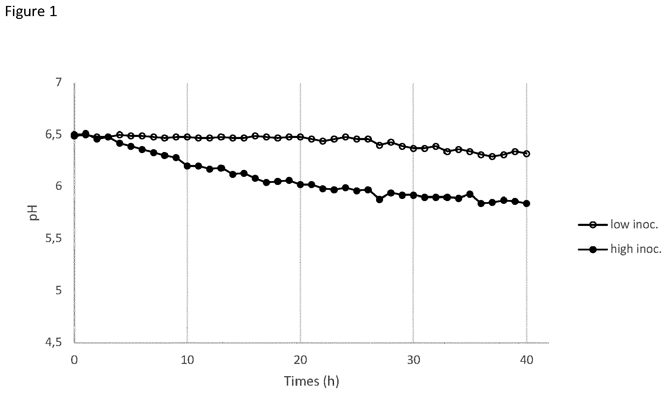

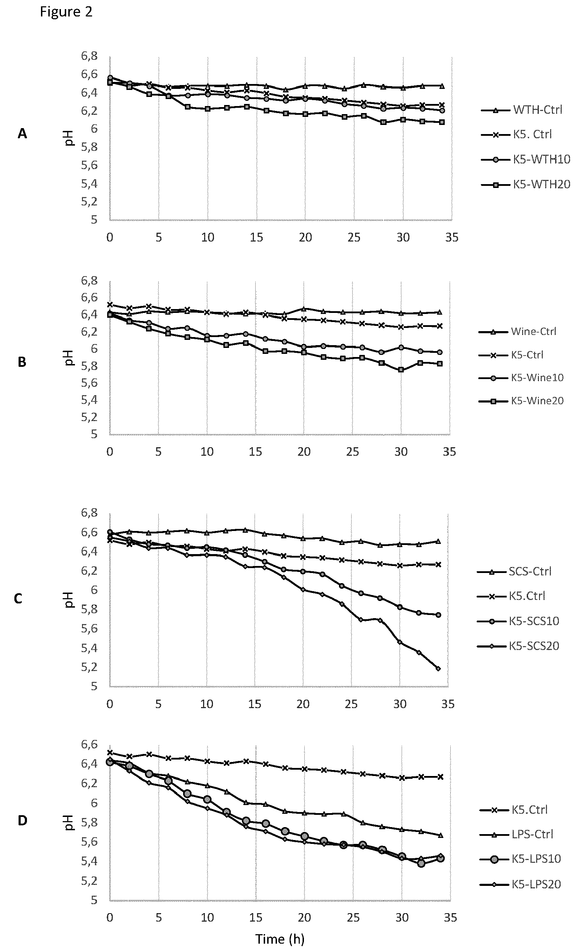

Quite recently *L. kefiranofaciens* and *L. kefiri* have been linked with various interesting health promoting benefits and it is believed they will be the next generation of food probiotics. However, one of the main technical disadvantages in the exploitation of these two important bacterial species of kefir grains and in kefir beverages and kefir derived products which are produced by these grains is their extremely slow growth and that their special needs for cultivation in the fermentation medium, especially pH value of the fermentation medium and supply of nutrients have to be meet due to their many metabolic restrictions e.g., in synthesis of amino acids and vitamins. Thus, in practice making their utilization not feasible on industrial scale. The inventors of the present invention could show that these two Lactobacilli species, individually and even in co-culture, are not able to ferment and acidify milk and meet the expectation of reducing the milk pH to a value of at least below 4.7 and below (cf. Figure 1 and 2). In addition to their slow growth, enumeration and quantification of these species using the current analytical methods (e.g., plate counting of colonies) is not a fast and easy task, which further limits their application in industry. According to the present invention fermentation of the raw beverage source is the results of the growth and propagation of the microorganisms, they consume sugars, digest proteins and produce metabolites like ethanol, CO₂, organic acids among others.

In classical grain-based kefir nutritious needs to grow in the raw beverage source, are mostly provided by other yeasts and auxiliary bacteria. However, when utilizing starter cultures instead unless a suitable mixture of auxiliary bacteria is used which has not been fulfilled in the state of the art yet, the propagation of these two core species *(L. kefiranofaciens* and *L. kefiri)* will be inhibited, which - depending on the total fermentation time - will lead to an overgrow of the two lactobacilli by the auxiliary bacteria. This in the end may lead in the kefir-like fermented (end) product to very low numbers of *L. kefiranofaciens* and *L. kefiri* of at least below the cell number of the applied starting cell concentration (e.g., at least below 1* 10⁶ cells /mL, 1* 10⁵ cells /mL, 1* 10⁴ cells /mL or 1* 10³ cells /mL fermentation medium, when the starting cell concentration is ca. 1* 10⁶ cells /mL fermentation medium) on large scale approaches as used in the industry. Alternatively, it may even lead to a total absence of *L. kefiranofaciens* and *L. kefiri.* Former state of the art approaches may have underestimated such overgrow of fermentation microorganisms as it might happen with e.g., *L. kefiranofaciens* and *L. kefiri* as they do not generally apply methods to differentiate between different microbial fermentation species when performing cell number counting (if any cell counting is performed) in the produced kefir product.

There is therefore a need for providing an industrial method for kefir-grain free cultivation of the predominant kefir-related microorganism species *L. kefiranofaciens* and/or *L. kefiri* for making the production of a synthetic kefir feasible for industrial scale production reassembling the organoleptic and compositional characteristics of classical kefir more closely than currently available industrial kefir.

It is thus an object of the present invention to overcome or at least reduce at least one of the described disadvantages of the state of the art, especially to provide a reliable and effective method for kefir-grain free cultivation of kefir microorganism cultures comprising or consisting of *L. kefiranofaciens* and *L. kefiri* for the production of synthetic kefir-like fermented products.

The object of the present invention has been solved by the newly developed method as specified in claim 1 of the present invention.

In particular a method to produce a synthetic kefir-like fermentation product by bacterial strain from a raw beverage source is provided comprising the steps:
a. providing a row beverage source and providing as fermentation microorganism at least one *Lactobacillus* selected from the group consisting of: an isolated *Lactobacillus kefiranofaciens* strain, an isolated *Lactobacillus kefiri* strain, a purified culture of *Lactobacillus kefiranofaciens,* a purified culture of *Lactobacillus kefiri* and mixtures thereof and optionally providing at least one yeast strain derived from natural kefirs;
b. adding the fermentation microorganism of step a. to a raw beverage source to receive a fermentation medium;
c. applying conditions to facilitate growth and division of the fermentation microorganism to allow for fermentation of the fermentation medium;
d. receiving the synthetic kefir-like fermentation product.

The inventors of the present invention have advantageously and surprisingly found that by running the method of the present invention under conditions to facilitate growth and division of the fermentation microorganism that this leads to a stable high growth and division of the normally slow propagating *Lactobacillus kefiranofaciens* and *Lactobacillus kefiri* strains at levels to be usable at an industrial scale while at the same time avoiding an overgrow of other eventually applied microorganisms (auxiliary bacteria or yeast) which may be optionally co-cultured with at least one of these two core species.

The inventors of the present invention kept these slowly growing and metabolic restricted two bacterial strains *Lactobacillus kefiranofaciens* and *Lactobacillus kefiri* in the center of their work. Given these propagation restriction, there was the need to find the appropriate conditions to promote their growths in order to obtain a very high cell number of them in the final kefir-like fermented product. Achieving high numbers of these two bacteria, not only for the elevating of the health benefits is important, but also it is important in the process of bacterial aggregation and kefir-grain generation.

A "synthetic kefir-like fermentation product" according to the present invention is to be understood as a product which is derived by microbial fermentation utilizing microbial cell cultures, i.e., one which is not based on kefir-grain cultivation and fermentation (thus "synthetic"), which resembles organoleptic natural kefir produced by classical kefir-grains, especially by providing and containing *L. kefiranofaciens* and/or *L. kefiri* ("kefir-like fermentation product"). The term "kefir-like fermentation product" as used herein is not restricted to only kefir beverage itself but also various similar fermented products including not only fluid or pasty products but also gelatinous fermented products as e.g., soft yogurt and hard/ kerned yogurt. The synthetic kefir-like fermentation product may be also used as a pre-mixture or as a blend component for other beverage and/food related compositions, e.g., yogurt drinks, bakery goods (cake, cup cake, muffins, pop-cakes, etc.), cream fillings or toppings for bakery goods, ice cream etc..

*Lactobacillus kefiranofaciens* according to the present invention is to be understood as to include the subspecies of *kefirgranum* and/or *kefiranofaciens.*

According to an embodiment of the present invention in step a. an isolated *Lactobacillus kefiranofaciens* strain, an isolated *Lactobacillus kefiri* strain or mixtures thereof are used.

In case the fermentation microorganisms include at least one *Lactobacillus* of the group as specified in claim 1 and additionally at least one yeast strain it is possible that these applied fermentation microorganisms are all at once or only species-wise (and that optionally in fractions) either simultaneously or sequentially added to the raw beverage source. It is also possible that in case that the at least one *Lactobacillus* comprises *L. kefiranofaciens* and *L. kefiri,* that these two are added simultaneously followed by the sequential addition of the at least one yeast strain or that one of the *L. kefiranofaciens* and *L. kefiri* is added simultaneously with the at least one yeast strain and the remaining of the both *Lactobacillus* is added sequentially.

An "isolated strain" or a "purified cell culture thereof" are received by conventional isolation, cell cultivation and purification techniques well known by a skilled person.

For *in vitro* cell cultivation of Lactic acid bacteria (including *Lactobacillus, Lentilactobacillus, Lactococcus, Leuconostoc* etc.) normally a medium called MRS (a non-well defined medium) is used which contains various nutritious compounds like meat extract and yeast extract, as these bacteria are not able to synthesis all their needs. *L. kefiranofaciens* and *L. kefiri* are known to have metabolic restrictions, especially with respect of synthesis of the nutrients like amino acids and vitamins. Thus, according to an embodiment of the method of the present invention the "conditions to facilitate growth and division of step c." comprise that the fermentation medium is supplemented with a pre-fermented acidified raw beverage source derived by microbial fermentation and/or by enzymatic treatment comprising free amino acids and/or free oligopeptides. According to another embodiment of the method of the present invention the raw beverage source is supplemented with a pre-fermented acidified raw beverage source derived by microbial fermentation and/or by enzymatic treatment comprising free amino acids and/or free oligopeptides. Free amino acids and amino acid derivatives comprise all amino acids important for the propagation of the *L. kefiranofaciens* and *L. kefiri,* including e.g., glutamic acid, asparagine, proline, tyrosine, cysteine and valine. Free oligopeptides comprise all free oligonucleotides important for the propagation of the *L. kefiranofaciens* and *L. kefiri,* including D-alanyl-D-alanine, caseinomacropeptide. Also, other nutrients may be supplied as e.g., vitamins, purines and purine nucleotides and fatty acids. Vitamins comprise pantothenic acid, biotin, folic acid, riboflavin, niacin, pyridoxine, ascorbic acid (vitamin C). Purines and purine nucleotides comprise AMP, GMP, XMP and their derivatives including c-di-AMP. Free fatty acids comprise Tween 20, Tween 80, and Octadecenoic acids such as oleic acid, elaidic acid, cis-vaccenic acid and vaccenic acid. These nutrients from a general point of view function positively for improving the growth, vitality, viability and survival during the cultivation and storage of the kefir-like fermentation product. The pre-fermented acidified raw beverage source may be added to the fermentation medium comprising the at least one *Lactobacillus* all at once or continuously, e.g. drop-wise. This is performed in amounts that are suitable to facilitate the envisioned propagation and reported beneficial propagation benefits (cell number increase) of the at least one *Lactobacillus* while reducing the pH at the envisioned level within under 24 hours of incubation of the raw beverage source comprising the at least one *Lactobacillus* (i.e., fermentation medium). E.g., if the pre-fermented acidified raw beverage source is added to the fermentation medium comprising the at least one *Lactobacillus* all at once the amount of the pre-fermented acidified raw beverage source is in the range of 1% v/v to 25% v/v, 3% v/v to 15% v/v or 3% v/v to 8% v/v of the total fermentation medium volume.

The enzymatic treatment may include the utilization of added proteases and/or peptidases, and carbohydrate-digesting enzymes as e.g., lactase or lipases. Suitable proteases, peptidases, carbohydrate-digesting enzymes, and lipases of the state of the art are well known to an artisan. In an alternative embodiment of the present invention the conditions to facilitate growth and division of step c. comprise that suitable proteases, peptidases, carbohydrate-digesting enzymes, and lipases as stated above may be directly added to the raw beverage source of step a. or to the fermentation medium of step b.

The pre-fermented acidified raw beverage source derived from microbial fermentation may be received by inoculate a raw beverage source with at least one isolated auxiliary bacterial strain or purified culture thereof derived from natural kefirs selected from the group consisting of: *Lactobacillus spp., Lentilactobacillus* spp., *Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobactersyzygii, Bifidobacterium* spp., *Enterococcus faecium, Enterococcus faecalis,* or mixtures thereof.

In general, in some or all embodiments utilizing the aforementioned at least one (optional) yeast strain or an isolated strain or purified culture thereof derived from natural kefirs, it maybe preferred to include at least one lactose-hydrolyzing yeast and additionally at least one or at least two lactose-unhydrolyzing yeast. Lactose-hydrolyzing yeast according to the present invention hydrolyze lactose to glucose and galactose and include *Kluyveromyces marxianus* and *Dekkera anomala.* Lactose-unhydrolyzing yeast according to the present invention are selected from the group consisting of: *Saccharomyces cerevisiae, Kazachstania unispora, Kazachstania turicensis, Kazachstania exigua, Debaryomyces hansenii, Saccharomyces boulardii* or mixtures thereof.

The community/consortium of auxiliary bacteria or auxiliary bacteria and yeasts have to fulfil the pH reduction requirement, i.e., the auxiliary bacteria or auxiliary bacteria and yeasts have to be active to enable a pH reduction of the raw beverage source to a low pH value, preferably of at least 4.7 and below. Following acidification / fermentation of the raw beverage source the complete pre-fermented acidified raw beverage source or a sample thereof is added to another raw beverage source comprising cultures of *L. kefiranofaciens* and/or *L. kefiri.* The inventors of the present invention have found out that the following isolated auxiliary bacterial strain or purified culture thereof derived from natural kefirs namely *Lactococcus lactis, Acetobacter orientalis and Leuconostoc mesenteroides* advantageously deliver a pre-fermented acidified raw beverage source leading to an at least 7.5-fold, at least 10-fold or an at least 15-fold increase of the starting cell concentration of the at least one Lactobacillus when applied under 24h or 48h of fermentation when used alone or in mixtures of at least two.

The pre-fermented acidified raw beverage source fraction may be either cell-free or cell-containing. It is also possible that the raw beverage source used to produce the pre-fermented acidified raw beverage source and the raw beverage source comprising cultures of *L. kefiranofaciens* and/or *L. kefiri* may be fractions of the same raw beverage source entity (e.g., both are derived from the same cow's milk sample) or at least from the same type of beverage source (e.g., both are derived from cow's milk) or alternatively both are derived from two different sources (e.g., one derives from cow's milk and the other one derives from goat's milk). A "cell free" pre-fermented acidified raw beverage source fraction may be received by appropriate methods to separate the fermentation microorganisms (cells) from the rest of the pre-fermented acidified raw beverage source fraction (medium) well known to the artisan. Theses cell separation methods may include centrifugation, filtration techniques as e.g., microfiltration or combinations thereof.

"Cell containing" according to the present invention means that the pre-fermented acidified raw beverage source comprises at least one microorganism of the at least one fermentation microorganisms which was added to inoculate the raw beverage source to receive the pre-fermented acidified raw beverage source and which pertains in the resulted pre-fermented acidified raw beverage source. This at least one microorganism may be at least one isolated auxiliary bacterial strain or purified culture thereof derived from natural kefirs selected from the group consisting of: *Lactobacillus spp., Lentilactobacillus* spp., *Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobacter syzygii, Bifidobacterium* spp., *Enterococcus faecium, Enterococcus faecalis,* or mixtures thereof. Additionally, or alternatively the pre-fermented acidified raw beverage source fraction may contain at least one yeast strain or an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof.

By utilizing a pre-fermented acidified raw beverage source, comprising cells of the at least one microorganism (auxiliary bacteria and/or yeast) of the present invention which is after (pre-)fermentation and acidification added to the culture of at least one *Lactobacillus* species (*L*. *kefiranofaciens* and *L. kefiri)* in a raw beverage source (fermentation medium), wherein the culture of the at least one *Lactobacillus* species was optionally co-cultured with at least one yeast may significantly reduce the risk of an overgrow of the at least one *Lactobacillus* species *(L. kefiranofaciens* and *L. kefiri).*

This reduction of a potential overgrowth of at least one *Lactobacillus* species *(L. kefiranofaciens* and *L. kefiri)* may be further significantly reduced by applying a "cell free" or "centrifuged and thus cell reduced" pre-fermented acidified raw beverage source.

According to another embodiment of the method of the present invention, the cell numbers of the at least one *Lactobacillus* are discontinuously (time-wise, e.g., at certain time points) or continuously monitored and optionally discontinuously (time-wise, e.g., at certain time points) or continuously regulated. In this context, "controlling" is understood in the general common meaning of keeping under constant monitoring the parameters related to the culture and essentially measuring said parameters or status indicators, using common methodologies and measuring instrumentation known in the art. Since it might not be sufficient to keep under constant monitoring this parameter of the culture, therefore according to an additional embodiment of the present invention comprise in particular regulating the cell numbers of the at least one *Lactobacillus* continuously. In the understanding of the present invention, "regulating of the cell numbers" is intended as actively promoting the propagation of the at least one *Lactobacillus* (cell growth and division) until a certain given value for that parameter is reached by using appropriate means to do so. E.g., such a regulation can be realized by changing the fermentation medium at least partly or by adding at least one nutrient, which triggers cell division and cell growth at least of the at least one *Lactobacillus.* Nutrients, which trigger cell growth and cell division are well known by an artisan and include the addition or the increase of nutrients e.g., vitamins, amino acids, fatty acids, mono-, di-, oligo- and polysaccharides or precursors of all of the aforementioned and cell growth factors.

A "given value" according to the present invention may be a defined value with given tolerances, tolerances within the measurements system or tolerances due to the variability within the culture or due to the culture diversity or it may be a range of suitable values.

According to a further embodiment of the method of the present invention the conditions to facilitate growth and division of step c. comprise keeping the temperature in a range from 16 °C and 37 °C, 16°C and 30°C or 18°C to 25°C. Especially when the at least one *Lactobacillus* is co-cultured with auxiliary bacterial strains and/or yeast keeping the temperature in a range from 16 °C and 37 °C and 18°C and 30°C or 20°C to 28°C are preferred.

According to a further embodiment of the method of the present invention, the method further comprises:
further adding to the raw beverage source or to the fermentation medium at least one isolated auxiliary bacterial strain or purified culture thereof derived from natural kefirs selected from the group consisting of: *Lactobacillus spp., Lentilactobacillus* spp., *Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobacter syzygii, Bifidobacterium* spp., *Enterococcus faecium, Enterococcus faecalis,* or mixtures thereof.

*Leuconostoc mesenteroides* according to the present invention is to be understood as to include the strains LgPr3 and/or Lg2a and the subspecies of *cremoris, dextranicum, mesenteroides* and *jonggajibkim.*

*Lactococcus lactis* according to the present invention is to be understood as to include the strains DC103 and/or NZ9000 and the subspecies of *cremoris, lactis* and *lactis* biovar. *diacetylactis.*

Acetic acid produced by Acetobacter has been shown for certain bacterial strains and yeast strains as a growth promoting factor. In low doses it generally promotes microbial cell growth whereas in high doses it reduces or even blocks concentration dependently the microbial cell growth. According to some or all embodiment of the method of the present invention utilizing at least one isolated auxiliary bacterial strain or purified culture thereof derived from natural kefirs at least one Acetobacter is comprised selected from the group consisting of: *Acetobacter orientalis, Acetobacter fabarum, Acetobacter okinawensis, Acetobacter lovaniensis, Acetobacter syzygii* or mixtures thereof, preferably *Acetobacter orientalis and* mixtures with the aforementioned.

According to an embodiment of the method of the present invention, the at least one optional yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof. According to an embodiment the yeast strain is added obligate.

According to a further embodiment of the method of the present invention the conditions to facilitate growth and division of step c. comprise keeping the temperature in a range from 16 °C and 37 °C, 16°C and 30°C or 18°C to 25°C. Especially when the at least one *Lactobacillus* is co-cultured with auxiliary bacterial strains and/or yeast keeping the temperature in a range from 16 °C and 37 °C and 18°C and 30°C or 20°C to 28°C are preferred. The inventors of the present invention have found temperatures in a range from 18°C to 25°C work the best when there is no yeast added.

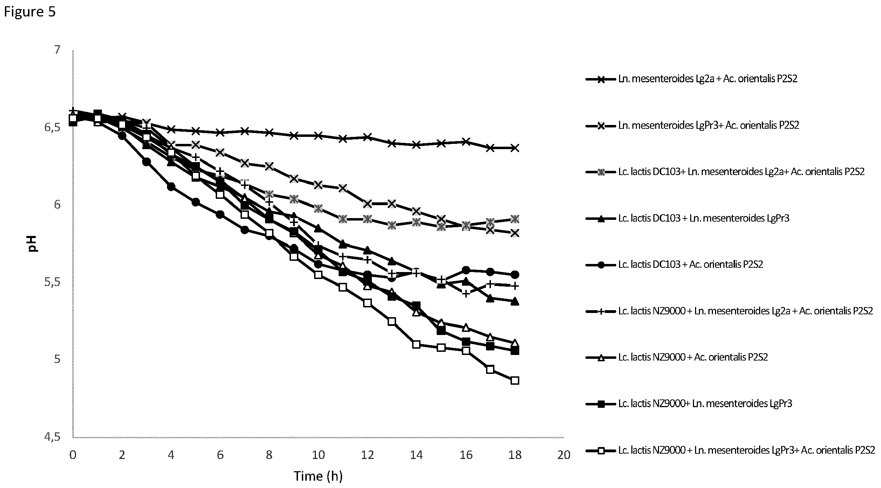

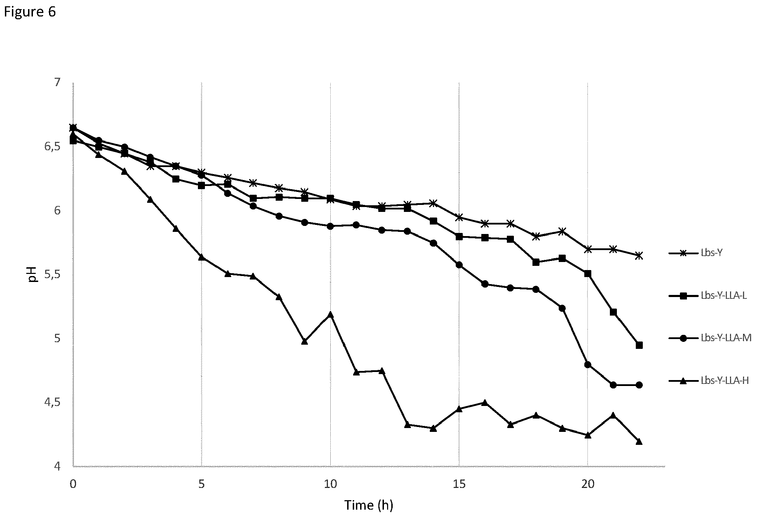

According to a further embodiment of the method of the present invention the method additionally comprises:
further adding to the raw beverage source or to the fermentation medium comprising the at least one Lactobacillus all of the following isolated auxiliary bacterial strain or purified culture thereof derived from natural kefirs: *Lactococcus lactis and Leuconostoc mesenteroides.* Such a co-culture of the at least one Lactobacillus and *Lactococcus lactis and Leuconostoc mesenteroides* advantageously reduces the pH of the raw beverage source to a pH of at least 4.7 and below within under 24h or 48h of fermentation (cf. FIG. 5 and FIG. 6) and leading to an at least 7.5-fold, at least 10-fold or an at least 15-fold increase of the starting cell concentration of the at least one *Lactobacillus* within under 24h or 48h of fermentation (cf. FIG. 6). The inventors of the present invention have found out that especially these two auxiliary bacteria when co-cultured with the at least one Lactobacillus have the reported beneficial propagation benefits (cell number increase) of the at least one *Lactobacillus* while reducing the pH at the envisioned level within under 24 hours or 48h of incubation. They build the minimum of auxiliary bacteria co-cultured with the at least one *Lactobacillus.*

According to another further embodiment of the method of the present invention the method additionally comprises:
further adding to the raw beverage source or to the fermentation medium comprising the at least one *Lactobacillus* all of the following isolated auxiliary bacterial strain or purified culture thereof derived from natural kefirs: *Lactococcus lactis* and *Leuconostoc mesenteroides* and adding additionally at least one yeast strain, wherein the yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof. The further addition of at least one yeast strain to such a co-culture of the at least one Lactobacillus and *Lactococcus lactis* and *Leuconostoc mesenteroides* advantageously even more efficient reduces the pH of the raw beverage source to a pH of below 4.7 within under 24h of fermentation (data not shown). The further addition of two and more of the listed yeast further synergistically increase this beneficial effect. When using co-cultures of *L. kefiranofaciens* and *L. kefiri* together with *Lactococcus lactis, Acetobacter orientalis* and *Leuconostoc mesenteroides* and mixtures of three yeast strains, namely *Saccharomyces cerevisiae, Kazachstania unispora,* and *Kluyveromyces marxianus* the synergistic and beneficial effect was also shown (cf. FIG. 6).

In case the fermentation microorganisms include at least one *Lactobacillus* of the group as specified in claim 1 and additionally at least one yeast strain and additionally at least one auxiliary bacteria strain it is possible that these applied fermentation microorganisms are all at once or only species-wise (and that optionally in fractions) either simultaneously or sequentially added to the raw beverage source.

Without being bound by that theory the non-"*L kefiranofaciens*/*L. kefiri"* microorganisms can be roughly put in 2 categories;
1) auxiliary bacteria/yeasts that are supposed to function as "auxiliary", which provide pH reduction and growth requirement of the *L. kefiranofaciens*/*L. kefiri,* like *Ln. mesenteroides, S. cerevisiae,* and
2) auxiliary bacteria/yeasts that not necessarily supply other microorganisms but **they can add health value** to the kefir-like fermented product, as probiotic activity, production of some special metabolites, which probably *Lacticaseibacillus rhamnosus, Bifidobacterium spp.* can be assigned into this category.

The community/consortium of auxiliary bacteria or auxiliary bacteria and yeasts which are co-cultured with *L. kefiranofaciens* and/or *L. kefiri* have to fulfil the pH reduction requirement, i.e., the auxiliary bacteria or auxiliary bacteria and yeasts have to be active to enable a pH reduction of the raw beverage source, e.g., milk before 24 h or before 48 h post-inoculation with them (fermentation) to a low pH value, preferably of at least 4.7 and below, which is a major indication of good growth and a quality criteria of fermented sour products, e.g. sour milk products.

As stated above the core bacteria *(L. kefiranofaciens* and *L. kefiri),* when are inoculated in a raw beverage source, e.g., milk, individually or both together, have very poor growth ability- according to the best knowledge of the inventors - due to their many metabolic restrictions in synthesis of especially amino acids and vitamins. The inventors of the present invention have advantageously and surprisingly found out that co-cultivation of at least one of the slow growing bacteria *L*. *kefiranofaciens* and *L. kefiri* in a raw beverage source, e.g., milk by enabling their propagation through mixed cultivation with other specific auxiliary bacteria and/or yeasts which are derived from natural grain-based kefirs. These auxiliary bacteria and/or yeasts provide *L. kefiranofaciens* and *L. kefiri* with their missing nutrients (growth factors, vitamins, free amino acids, fatty acids and precursors of the aforementioned) thus promoting their propagation. Additionally, yeasts provide kefir-like flavor to the (end) products; in embodiments without yeast this function may be provided by additionally adding commercially available yeast extract. This approach is found to be very critical as in the selection of other strains this fact has be taken into account that auxiliary microorganisms must not inhibit the growth of *L. kefiranofaciens* and *L. kefiri* and must not overgrow the *L. kefiranofaciens* and *L. kefiri.* The inventors of the present invention have found that when *L. kefiranofaciens* and/or *L. kefiri* are co-cultured with at least one isolated auxiliary bacterial strain or purified culture thereof derived from natural kefirs and optionally with at least one yeast strain derived from natural kefirs that this leads to controlled mixed co-cultures of fermentation microorganisms, capable to promote the propagation of *L. kefiranofaciens* and *L. kefiri* individually and both together. Adding each new additional microorganism can add new functionalities (health properties, technical change in technical parameters during production (e.g., time of fermentation, O₂/CO₂ content, survivability of strains, polysaccharide production, etc.) and organoleptic characteristics) to previous combination, and the specific microbial strain has to be chosen carefully to not suppress the main functions of *L. kefiranofaciens* and/or *L. kefiri.*

According to an embodiment of the method of the present invention, step c. further comprises: monitoring and, optionally continuously regulating the pH value in step c. to be at a given range of 3.5 to 5.5, preferably 4.1 to 5.0, more preferably 4.1 to 4.7. A monitoring and regulating of the pH can have a benefit for the control of the growth of *L. kefiranofaciens* and *L. kefiri both* alone, in combination or in co-culture (each alone or both) with the auxiliary bacteria and/or yeast.

Since it might not be sufficient to keep the pH value under constant monitoring this parameter of the culture, therefore according to an additional embodiment of the present invention comprise in particular regulating the pH in the culture continuously. In the understanding of the present invention, "regulating" is intended as actively influence the pH to be at a given value or a given value span for that parameter by using appropriate means to do so.

According to an embodiment of the method of the present invention, step b. or c. further comprises:
adding at least one scaffold material to the raw beverage source or to the fermentation medium, wherein the at least one scaffold material is an exopolysaccharide selected from the group consisting of: kefiran, dextran, alternan, galactan, fructan, mutan, levan, reuteran, pullulan, curdlan and xanthan;
or is a natural polymer selected from the group consisting of: collagen and chitosan or is a synthetic polymer selected from the group consisting of: poly (ε- caprolactone) (PCL), poly (lactic acid) (PLA), poly (glycolic acid) (PGA), poly (3 hydroxybutyrate-co-3-hydroxyvalerate) (PHBV), and poly (lactic-co-glycolic acid) (PLGA).

According to the present invention exopolysaccharides, and synthetic polymers are to be understood to comprise a molecular weight of 1.0 × 10¹ to 1.0 × 10⁶ kDa, preferably 1.0 × 10² to 1.0 × 10⁵ kDa, more preferably of 1.0 × 10³ to 1.0 × 10⁴ kDa.

According to the present invention natural polymers collagen and chitosan and hydrolysed collagen and chitosan have a molecular weight of 3 to 400 kDa, preferably 5 to 300 kDa, more preferably of 100 - 200 kDa.

According to the present invention synthetic polymers have a molecular weight of 2 to 500 kDa, preferably 2 to 400 kDa, more preferably of 2 - 300 kDa.

According to a preferred embodiment of the method of the present invention the at least one scaffold material is kefiran.

According to an embodiment of the method of the present invention, the raw beverage source is derived from mammal's milk and selected from the group consisting of: cow milk, goat milk, sheep milk, horse milk, buffalo milk.

According to an embodiment of the method of the present invention, the at least one *Lactobacillus* of step a is added at a starting cell concentration of at least 1* 10⁶ cells/mL to 1* 10⁹ cells/mL fermentation medium and wherein the at least one *Lactobacillus* is grown by applying conditions of step c. to an end cell concentration of at least 1* 10⁷ cells/mL to 1* 10⁹ cells/mL kefir-like fermentation product preferably within under 24h or 48h of fermentation.

According to an embodiment of the method of the present invention, the at least one *Lactobacillus* of step a is added at a starting cell concentration of at least 1* 10⁶ cells/mL to 1* 10⁹ cells/mL fermentation medium or 1* 10⁷ cells/mL to 1* 10⁸ cells/mL fermentation medium and wherein the at least one *Lactobacillus* is grown by applying conditions of step c. to an end cell concentration in the kefir-like fermentation product, which is advantageously at least 7.5-fold higher, at least 10-fold higher or at least 15-fold higher than the starting cell concentration preferably within under 24h or 48h of fermentation. I.e., when, e.g., a starting cell concentration of 1* 10⁶ cells/mL fermentation medium is used the at least one *Lactobacillus* is grown up to at least 1* 10⁷ cells/mL (10-fold increase) more preferred up to at least 1* 10⁸ cells/mL, most preferred up to at least 1* 10⁹ cells/mL kefir-like fermentation product within under 24h (e.g., 18h, 22h or 23h) or within under 48h (e.g., 30h, 35h or 43h). Thus, given the inventive method of the present invention cell numbers of the two core bacteria *L. kefiranofaciens* and *L. kefiri* remarkedly increased and could be reached by culturing the two core bacteria that resembles the cell number of these *Lactobacilli* strains in natural grain-based kefir beverages, i.e., when these *bacterial* strains are grown and multiply when cultured by utilizing kefir-grains. As stated above for *L. kefiranofaciens:* a minimum of 1* 10⁸ cells/ mL of classical kefir beverage was found by utilizing natural grain-based kefir production; *L. kefiri:* a minimum of 1* 10⁷ cells/ mL of classical kefir beverage was found by utilizing natural grain-based kefir production (cf. Nejati, et al. 2020).

According to an embodiment of the method of the present invention the at least one isolated auxiliary bacterial strain or purified culture thereof is added at a starting cell concentration of 1* 10⁴ cells/mL to 1* 10⁸ cells/mL cells/mL fermentation medium and wherein the at least one auxiliary bacterial strain is grown by applying conditions of step c. to an end cell concentration, which is at least 10-fold up to 1000-fold higher than the starting cell concentration preferably within under 24h or48h of fermentation.

According to an embodiment of the method of the present invention the at least one optional yeast strain is added together with the culture of the two core bacteria or the two core bacteria and the auxiliary bacterial strain at a starting cell concentration of 1* 10³ cells/mL to 1* 10⁶ cells/mL fermentation medium and wherein the at least one yeast strain is grown by applying conditions of step c. to an end cell concentration, which is at least 7.5 or at least 10 -fold higher than the starting cell concentration preferably within under 24h or 48h of fermentation.

Co-cultures of *L. kefiranofaciens* and *L. kefiri* together with *Lactococcus lactis, Acetobacter orientalis* and *Leuconostoc mesenteroides and* mixtures of three yeast strains, namely *Saccharomyces cerevisiae, Kazachstania unispora,* and *Dekkera anomala* showed a beneficial increase of *L. kefiranofaciens* and *L. kefiri* cell concentrations under 24 h of fermentation (22h) in a manner depending on the concentration of the auxiliary bacteria strain and yeast strains. A high number of highly metabolic active auxiliary bacterial strains and yeast strains was leading to a fast drop in pH which was associated with a massive reduction of the cell concentration increase of *L. kefiranofaciens and L. kefiri* (cf. FIG. 6). Thus, indicating not only a pH drop in short time but also - without being bound by that theory - an underlying overgrow of the two lactobacilli by the utilized propagating auxiliary bacterial strains and yeast strains. This in the end may led in the kefir-like fermented (end) product to very low numbers of *L. kefiranofaciens* and *L. kefiri* as measured. Thus, according to an embodiment of the present invention, the at least one *Lactobacillus* of step a is added at a starting cell concentration of at least 1* 10⁶ cells/mL to 1* 10⁹ cells/mL fermentation medium or 1* 10⁷ cells/mL to 1* 10⁸ cells/mL fermentation medium and wherein the at least one *Lactobacillus* is grown by applying conditions of step c. to an end cell concentration in the kefir-like fermentation product, which is advantageously at least 7.5-fold higher, at least 10-fold higher or at least 15-fold higher than the starting cell concentration preferably within under 24h or 48h of fermentation. The method additionally comprises:
further adding to the raw beverage source or to the fermentation medium comprising the at least one *Lactobacillus* all of the following isolated auxiliary bacterial strain or purified culture thereof derived from natural kefirs: *Lactococcus lactis* and *Leuconostoc mesenteroides* and adding additionally at least one yeast strain, wherein the yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof, preferably *Dekkera anomala, Saccharomyces cerevisiae, Kazachstania unispora* or mixtures thereof. And, wherein the auxiliary bacterial strain or purified culture thereof is added at a starting cell concentration of 1* 10⁴ cells/mL to 1* 10⁵ cells/mL. The addition of this auxiliary bacterial strain and yeast strain subset in that starting concentration advantageously reduces the pH of the raw beverage source to a pH of below 4.7 within under 24h but not too harsh per time (fast). I.e., the *L. kefiranofaciens and L. kefiri* are promisingly not inhibited by harsh high pH drop nor high concentration of metabolic active and propagating auxiliary bacterial strains and/or yeast strains competing with *L. kefiranofaciens and L. kefiri* for nutrients and overgrowing *L. kefiranofaciens and L. kefiri* (cf. FIG. 6). Indicating that such conditions lead to a favourable balanced growth and propagation of *L. kefiranofaciens and L. kefiri* to high cell concentrations in the kefir-like fermentation product which is at least 7.5-fold higher, at least 10-fold higher or at least 15-fold higher than the starting cell concentration preferably within under 24h or 48h of fermentation.

Appropriate methods to species-unspecifically calculate cell numbers of microorganisms in monoculture (e.g., bacteria-/yeast- species specific starting concentration) are well known to the artisan and do include unspecific quantitative real time PCR (without probe) or may include classical techniques as flow cytometry utilizing species-specific markers, plate count method (resulting in Colony forming units, CFU). Appropriate methods to species-specifically calculate cell numbers of microorganisms in the mixed cultures e.g., of the fermentation medium or the synthetic kefir-like fermentation product as used in the present invention (bacteria-/yeast-species specific concentration) are well known to the artisan and do include species-specific primer-probe sets as described in (Nejati, et al. 2020) or may include classical techniques as flow cytometry utilizing species-specific markers.

According to an embodiment of the method of the present invention the end cell concentration of the at least one *Lactobacillus* and/or the at least one isolated auxiliary bacterial strain or purified culture thereof in the kefir-like fermentation product is reached within 15h, 18h, 20h, 22h, 23h, 30h, 35h or 47h after adding the fermentation microorganism to the fermentation medium. The inventors of the present invention have advantageously found that the cultivation of the at least one *Lactobacillus* together with at least one auxiliary bacterial strain leads to a reduction in fermentation time to less than 24 hours compared to when using classical kefir-grain fermentation which needs 24 h and above. An incubation time longer than under 24h of e.g., 30h, 36h or 48h maybe performed to increase the aimed fermentation result with respect to end cell numbers and organoleptic properties (e.g., consistency, taste etc.) of the kefir-like fermentation product. By the method as presented by Nejati, et al. 2020 it was possible to precisely enumerate the two core bacteria *L. kefiranofaciens* and *L. kefiri* as well as the auxiliary bacteria and yeast during the whole period of fermentation, thus allowing for accurate monitoring of the propagation of each single species during time. This species-specific cell quantification method thus positively enables to monitor species-specific cell propagation during fermentation. Based on this finding it is also possible to actively promote and strengthen cell growth and division of the fermentation microorganisms in a situation dependent manner, e.g., if the cell growth is regarded to be too slow to reach the aimed cell numbers in the envisioned fermentation time period of under 24h or under 48h. This by regulation of growth and division signals as e.g., the supply of growth factors or nutrients (e.g., vitamins, amino acids, fatty acids, mono-, di-, oligo- and polysaccharides or precursors of all of the aforementioned). Growth factors and nutrients which support the growth and division of fermentation microorganisms are well known to an artisan and herewith incorporated. Another strategy may be to facilitate growth and division in step c. by supplementing the fermentation medium with a pre-fermented acidified raw beverage source derived by microbial fermentation and/or by enzymatic treatment comprising free amino acids and/or free oligopeptides with the characteristics as described above.

According to another aspect of the present invention a microbial composition is provided to produce a synthetic kefir-like fermented product comprising as fermentation microorganism:
a. at least one *Lactobacillus* of the group consisting of an isolated *Lactobacillus kefiranofaciens* strain, an isolated *Lactobacillus kefiri* strain, a purified culture of *Lactobacillus kefiranofaciens,* a purified culture of *Lactobacillus kefiri* and mixtures thereof; and
b. optional at least one yeast strain, wherein the at least one yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof.

The invented microbial compositions resemble the microbial consortia of the milk grain-based kefir beverage and can be regarded as authentic artificial starter cultures for kefir production which has the benefit to be commercialized on a large industrial scale. This aspect of the invention is about to deliver a composition of artificial/synthetic kefir starter cultures with the most authentic microbial structure of natural kefir grains.

According to an embodiment of the microbial composition of the present invention the fermentation microorganism further comprise at least one auxiliary bacterial strain or purified culture thereof derived from natural kefirs selected from the group consisting of: *Lactobacillus spp., Lentilactobacillus* spp., *Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobacter syzygii, Bifidobacterium* spp., *Enterococcus faecium, Enterococcus faecalis,* or mixtures thereof.

According to another subject of the present invention a synthetic kefir-like fermented product is provided comprising as fermentation microorganism:
a. at least one *Lactobacillus* of the group comprising an isolated *Lactobacillus kefiranofaciens* strain, an isolated *Lactobacillus kefiri* strain, a purified culture of *Lactobacillus kefiranofaciens,* a purified culture of *Lactobacillus kefiri* and mixtures thereof; and
b. optionally at least one yeast strain,
wherein the at least one yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof.

Fermented raw beverage source formulations enriched with these two bacteria species will deliver several health supporting benefits upon consumption as food, feed or nutritional supplements.

According to an embodiment of the synthetic kefir-like fermented product the fermentation microorganism further comprise at least one auxiliary bacterial strain or purified culture thereof derived from natural kefirs selected from the group consisting of: *Lactobacillus spp., Lentilactobacillus* spp., *Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobacter syzygii, Bifidobacterium* spp., *Enterococcus faecium, Enterococcus faecalis,* or mixtures thereof.

According to a further embodiment of the synthetic kefir -like fermented product the kefir-like fermented product is a kefir-like fermented mammal's milk product selected from the group consisting of: cow milk, goat milk, sheep milk, horse milk, buffalo milk.

The milk fermented by using the mentioned microbial composition has several health supporting benefits, including:
- Prebiotic activity if the kefir-like fermented product comprises *L. kefiranofaciens: L. kefiranofaciens* is the only producer of the exopolysaccharide kefiran, which in addition to prebiotic functionality, has been linked to several other biofunctionalities such as anti-tumor activity
- Elevated probiotic activity: combination of several complementary microorganisms improves probiotic activities in comparison to single-species cultures, e.g., *L. kefiranofaciens* carries several genes important for bile acids degradation, that can support other cultures in co-cultivation to increase their population in the human intestine. Furthermore, the presence of different exopolysaccharides protects probiotic organisms against the harsh situations in the gastrointestinal tract
- Rich source of bioactive peptides (e.g., antihypertensive peptides, immunomodulatory peptides etc.) and microbial metabolites, such as butyrate derivatives (e.g. GABA).

According to another embodiment of the synthetic kefir-like fermented product the at least one *Lactobacillus (L. kefiranofaciens and*/*or L. kefiri)* has a concentration of at least 1* 10⁸ cells/mL kefir-like fermentation product.

According to another embodiment of the synthetic kefir-like fermented product wherein the at least one *Lactobacillus* comprises *L. kefiranofaciens* the amount of produced kefiran is at least 0.5 g/L, or at least 1.5 g/L, more preferred at least 2.0 g/L or at least 2.3 g/L most preferred at least 2.5 g/L kefir -like fermented product. Additionally, the synthetic kefir -like fermented product may comprise *L. kefiranofaciens* in a concentration of at least 1* 10⁸ cells/mL kefir-like fermentation product or may comprise both *L. kefiranofaciens* and *L. kefiri* each in a concentration of at least 1* 10⁸ cells/mL kefir-like fermentation product.

Accordingly, high concentrations of kefiran can be reached which may reach the level of kefiran in grain-based kefir beverage or may be positively even beyond that amount.

### References

Bourrie, Benjamin, Ju, Tingting, Fouhse, Janelle, Forgie, Andrew, Sergi, Consolato, Cotter, Paul, and Willing, Benjamin. (2021). Kefir microbial composition is a deciding factor in the physiological impact of kefir in a mouse model of obesity. Br. J. Nutr. 125(2), 129-138.
Nejati, Fatemeh, Junne, Stefan, Kurreck, Jens, and Neubauer, Peter. (2020). Quantification of Major Bacteria and Yeast Species in Kefir Consortia by Multiplex TaqMan qPCR. Front. Microbiol. 11 (June), 1291.
Rahbar Saadat, Yalda, Yari Khosroushahi, Ahmad, and Pourghassem Gargari, Bahram (2019). A comprehensive review of anticancer, immunomodulatory and health beneficial effects of the lactic acid bacteria exopolysaccharides. Carbohydr. Polym. Elsevier Ltd..
Slattery, Conor, Cotter, Paul D., and O'Toole, Paul W. (2019). Analysis of health benefits conferred by Lactobacillus species from kefir. Nutrients. MDPI AG.
Wang, Hao, Wang, Cuina, and Guo, Mingruo. (2020). Autogenic successions of bacteria and fungi in kefir grains from different origins when sub-cultured in goat milk. Food Res. Int. 138 (December), 109784.
Yokoi, Haruhiko, Watanabe, Takashi, Fuji, Yoshitsugu, Toba, Takahiro, and Adachi, Susumu. (1990). Isolation and characterization of polysaccharide-producing bacteria from kefir grains. J. Dairy Sci. 73, 1684-1689.

### Short description of the figures

**Figure 1****:**
   Microbial pH reduction activity over the fermentation time period of 40h of *L. kefiranofaciens and L. kefiri* in combination cultivated in low inoculum cell concentration (low inoc.) and high inoculum cell concentrations (high inoc.).
**Figure 2****:**
   A-D. Effect of different supplements on milk acidification *by L. kefiranofaciens* (K5). Milk was supplemented with 10 or 20 µl of, A) Wine-Tween 80-Hemin (WTH) mixture (WTH10 or WTH20), B) Wine (Wine10 or Wine20), C) S. *cerevisiae* supernatant (SCS; SCS10 or SCS20), and D) *Ln. mesenteroides* Lgpr3 and *Ac. orientalis* co-culture supernatant (LPS; LPS10 or LPS20). Supplemented milk without *L. kefiranofaciens* (X-Ctrl; X stands either for 20 µl of either WTH, Wine, SCS or LPS as indicated); and milk inoculated with *L. kefiranofaciens* without supplementation (K5-Ctrl) were analyzed as controls.
**Figure 3****:**
   A-E. Milk acidification by co-cultures of *L. kefiranofaciens* (K5), *Ln. mesenteroides* Lgpr3 and yeasts as described in Example 3. Yeast is one from the following species; S. *cerevisiae* DBVPG 10191, *Kz. unispora* DBVPG 6429, *Kz. turicensis* DBVPG 7206, *Kl. marxianus* DBVPG 6141, D. *anomala* DBVPG 10201.
**Figure 4****:**
   A-E. Milk acidification by co-cultures of *L. kefiranofaciens* K5 and *L. kefiri* 14 together (Lbs) and various combinations of yeast mixtures (Y1-Y5, see Table 1), *Ac. orientalis* P2S2 and *Ln. mesenteroides* Lgpr3 as described in Example 4.
**Figure 5****:**
   Milk acidification by combinations of various auxiliary species and strains as described in Example 5.
**Figure 6****:**
   Effect of initial concentration of axillary bacteria LLA (*Ln. mesenteroides, Lc. lactis* and *Ac*. *orientalis*) on the course of pH as well as the counts of *L. kefiranofaciens* and *L. kefiri* in the final fermented milk, Lbs-Y: mixed-culture of *L. kefiranofaciens* and *L. kefiri* and yeasts, co-cultured with low, medium and high initial concentrations of bacteria *Ln. mesenteroides, Lc. lactis* and *Ac. orientalis* (LLA-L, LLA-M and LLA-H, respectively) as described in Example 6.
**Figure 7****:**
   Effect of adding dextran on milk acidification by mixed cultures comprising of Lbs: *L*. *kefiranofaciens* and *L. kefiri,* Y: yeasts D. *anomala, S. cerevisiae and Kz. unispora,* and auxiliary bacteria (LL): *Lc. lactis* and *Ln. mesenteroides,* during 35 h fermentation as described in Example 7.

### Examples

The following examples illustrate viable ways of carrying out the described method as intended, without the intent of limiting the invention to said examples.

The following fermentation microorganisms were used in the Examples:

| *Core bacterial species* | *strain* |
|---|---|
| *L. kefiranofaciens* | K5 |
| *L. kefiri* | 14 |

### Auxiliary bacteria

| | |
|---|---|
| *Lc. lactis* | NZ9000 or DC103 |
| *Ln. mesenteroides* | Lgpr3 or Lg2a |
| *Ac. orientalis* | P2S2 |

### Yeasts

| | |
|---|---|
| *S. cerevisiae* | DBVPG 10191 |
| *Kz. turicensis* | DBVPG 7206 |
| *Kz. unispora* | DBVPG 6429 |
| *Kl. marxianus* | DBVPG 6141 |
| *D. anomala* | DBVPG 10201 |

### Example 1: co-cultures of L. kefiranofaciens and L. kefiri alone are not able to acidify and ferment the fermentation medium milk, indicating their slow propagation

Previous experiments of the research group of the Applicant succeeded in developing a fast and precise PCR based method for detection and quantification of eleven bacteria and yeasts of a kefir grain microbial community, including the two dominant bacteria: *L. kefiranofaciens* and *L. kefiri* as disclosed in Nejati, et al. 2020. Based on this method, it was possible for the first time to follow the growth of these species in mixed cultures comprising at least one of these two core bacterial species *(L. kefiranofaciens* / *L. kefiri*) with other microbial strains (yeasts and bacteria).

Health properties of any fermented products greatly is related to the microorganisms which are participating in the fermentation, which this has been mentioned for kefir (Bourrie et al. 2021), accordingly the currently available industrial kefirs can't have the same health properties as traditional (grain-based) kefir (there are many articles studied health benefits of traditional kefirs).It was the inventors aim to provide different combinations of microorganisms that can support
1) growth of *L. kefiranofaciens and L. kefiri* and
2) permit the pH reduction requirement in cow milk, to achieve the best authentic starter culture for kefir beverage production.

To start the pH decrease activity of the slow growing bacteria *L. kefiranofaciens* (both subspecies *kefiranofaciens* and *kefirgranums*) and *L. kefiri* in a raw beverage source cow milk was tested with different inoculated starting cell numbers. As shown in FIG. 1 low and high cell numbers (low inoc. and high inoc.) did not lead to a significant reduction in pH of the milk and showed the inability of these two lactobacilli species to decrease the milk pH in milk even after 40h of cultivation (fermentation). As stated before a reduction of the starting pH of the raw beverage source / fermentation medium to low pH values per time is a major indication of good growth. A pH of at least 4.7 and below within under 24h or 48h of fermentation is preferred and such a pH in the end product is a quality criteria of fermented sour products, e.g. sour milk products.

Thus, the results show that *L. kefiranofaciens* and *L. kefiri* are not able to acidify and thus propagate (grow and divide) in co-culture and thus to ferment the milk even after a long incubation for 40 h as the pH stays stably around the same pH value during the incubation time at low inoculum (around 5*10⁶ cells/mL for each bacterium). Even a high cell number of *L*. *kefiranofaciens* and *L. kefiri* (around 2*10⁷ cells/mL for each bacterium), only very slowly results in a slightly lower pH when compared with the starting pH of the fermentation medium (starting pH at 0h of incubation: ca. 6.65, pH at 40h of incubation: ca. 5.84). Thus, indicating that *L*. *kefiranofaciens* and *L. kefiri* need to be cultivated in consortia with other microbial species to promote their acid production (in cow milk lactose to lactic acid conversion mainly).

### Example 2. Acidification of supplemented milk by L. kefiranofaciens

As stated before, in classical kefir production using traditional grains, milk is acidified (pH reduced to below pH 4.7) during 24h-48h (depending on the grain : milk ratio). *L. kefiranofaciens* is the most dominant microorganism in kefir grain (followed by *L. kefiri),* therefore its ability in milk acidification was assessed in more detail. The effect supplementation of milk with wine, Tween 80 and hemin, was assessed and compared to the supplementation with the supernatant of *S*. *cerevisiae* as well as *Ln. mesenteroides* and *Ac. orientalis* grown in milk.

Cultures and materials: DSMZ.1253 medium was used to cultivate *L. kefiranofaciens.* In DSMZ.1253 medium the comprised components wine and hemin are supplementary ingredients to add to MRS medium) *L*. , and both were added according to DSMZ.1253 protocol. Tween 80 (lipid/fat source) and wine have been successfully applied by Yokoi et al (1990) in cultivation of *L. kefiranofaciens* in developed milk-based medium, and Tween 80 showed a very significant growth effect on Lactobacillus spp., e.g., *L. casei* in cheese model systems compared to other ingredients like yeast extract which is present in MRS medium. Accordingly, the effect of these 3 ingredients were assessed on *L. kefiranofaciens* acidification in milk. *L. kefiranofaciens* K5 was cultivated in MRS medium for 5 days in anerobic condition. S. *cerevisiae* DBVPG 10191 was cultivated in YPG medium for 1 day. Grown cultures of *Ln. mesenteroides* Lgpr3 and *Ac*. *orientalis* P2S2 were inoculated to milk (containing 0.2 % glucose) and incubated at 30 °C to clot the milk before the main fermentation.

Procedure: Milk fermentation was carried out at a temperature of 27 °C in transparent 96-well plate (Hydroplate HP96U; PreSens, Regensburg, Germany) which enables online pH monitoring (Hydroplate HP96U: A pH sensor is immobilized on the bottom of each well. The sensors are read out from the bottom side using a commercially available fluorescence reader and the approx. wavelengths 485/540 nm and 485/620 nm). Each well contains 260 µl milk (1.5% fat) supplemented with 10 or 20 µl of the followings: A) equal mixture of wine, Tween 80 (0.1% and 0.2% final concentrations in milk) and hemin (4 and 8 µg/mL final concentrations in milk), B) only wine, C) supernatant of S. *cerevisiae* DBVPG 10191 (centrifuged for 10 min, 4 °C, 8000 rpm) and D) supernatant of grown *Ln. mesenteroides* Lgpr3 and *Ac*. *orientalis* P2S2 in milk (centrifuged for 10 min, 4 °C, 8000 rpm). At the end, *L. kefiranofaciens* K5 was inoculated (7% w/w) in each well with supplement or without (water) as control.

FIG. 2 shows the drop of the starting pH in the experimental course of 33 h of milk fermentation. As it is shown *L. kefiranofaciens* K5 alone could only little acidify milk only from the starting pH 6.5 at 0 h of incubation to pH 6.27 at 33 h of incubation. The addition of wine, Tween 80 and hemin mixture was slightly helpful in further decreasing the pH (cf. FIG. 2 A). As addition of Tween 80 and hemin individually were not efficient in pH reduction by *L. kefiranofaciens* (results are not shown), two concentrations of wine were further studied (10 µl or 20 µl supplementation). Milk pH reduction by *L. kefiranofaciens* is positive correlated with wine concentration in milk (cf. FIG. 2 B). This means some wine components (e.g., sugars, minerals, remain microbial metabolites of wine fermentation, etc.) are necessary for milk acidification ability of *L. kefiranofaciens.*

However, as supplementation of milk with wine is not economical in industrial scale, in the following, the effect of three kefir-isolated microorganisms was analyzed. S. *cerevisiae* has shown synergistic effect on growth of *L. kefiranofaciens. S. cerevisiae* is, however, unable to grow in milk, and the supernatants of its growth in YPG was added to milk (S. cerevisiae supernatant (SCS); S. *cerevisiae* was grown for 1 day in YPG and then 10 or 20 microliter of its supernatant was added to milk). This supplementation shown significantly positive effect on *L. kefiranofaciens* acidification (cf. FIG. 2 C). *Ln. mesenteroides* and *Ac. orientalis* are other commonly isolated bacteria from kefir. The supernatant of milk fermented by these two bacteria showed significantly positive impact on milk acidification in combination with *L. kefiranofaciens.* The supernatant by themselves was also able to reduce milk pH, which is due to the activity and growth of the remaining bacterial cells from the supernatant (cf. FIG. 2 D).

Quite similar results were obtained when co-cultures of *L. kefiranofaciens* and *L. kefiri* and optional yeasts were supplemented with the cell-free supernatant of cultivations of auxiliary bacteria alone as described above which were preferably cultivated in cow's milk until they acidify the milk to pH 4.7 and below (data not shown).

### Example 3. Mixed cultures development 1: Milk acidification by L. kefiranofaciens co-cultured with a mixture of kefir-isolated bacteria and yeast

The object of this experiment is to assess the effect of mixture of two kefir-isolated microorganisms; *Ln. mesenteroides* and a yeast species: among S. *cerevisiae, Kz. turicensis, Kl. marxianus* and D. *anomala,* on milk acidification inoculated with *L. kefiranofaciens.*

Cultures: *L. kefiranofaciens* K5 was grown in MRS medium for 5 days at 28 °C in anaerobic condition. *Ln. mesenteroides* Lgpr3 was grown in MRS medium at 28 °C for 24 h in aerobic condition. Yeasts S. *cerevisiae* DBVPG 10191, *Kz. turicensis* DBVPG 7206, *Kz. unispora* DBVPG 6429, *Kl. marxianus* DBVPG 6141, D. *anomala* DBVPG 10201 were grown in YPG medium at 28 °C, for overnight (about 18h, for S. *cerevisiae, Kz. unispora* and *Kl. marxianus)* or 2 days *(Kz. turicensis* and D. *anomala*).

Procedure: Milk fermentation was carried out in 96-well plate (Hydroplate HP96U; PreSens, Regensburg, Germany) at a temperature of 27 °C. Each well contain 260 µl milk (1.5% fat) inoculated with 6 % v/v MRS-grown *L. kefiranofaciens* K5 alone or in combination with (1.8 %, v/v) of each yeast, or *Ln. mesenteroides* Lgpr3, or a mixture of thereof.

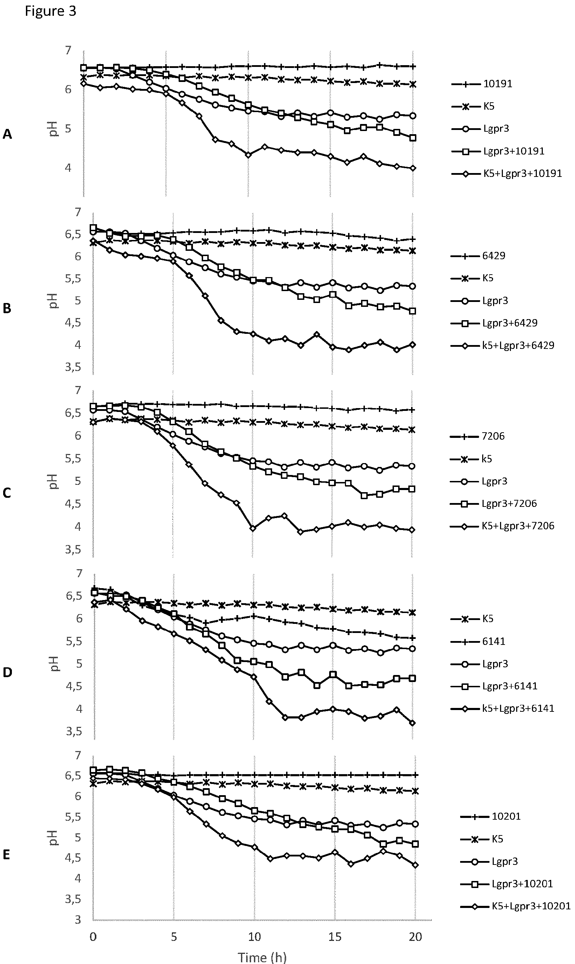

The results are depicted in FIG 3 and indicate that co-cultivation of *L. kefiranofaciens* with all combinations of *Ln. mesneteroides* and yeasts led to significantly faster milk pH reduction as compared to cultivation of single cultures in milk. Among applied yeast species, species *Kl. marxianus* and D. *anomala,* in the opposite of species S. *cerevisiae, Kz. unispora* and *Kz. turicensis,* are able to slightly decrease milk pH due to their ability of hydrolyzing milk's lactose. *Ln. mesenteroides* Lgpr3 was able to decrease milk pH to 5.30, which in combination with yeasts, the acidification ability was improved.

### Example 4. Mixed cultures development 2: Milk acidification by L. kefiranofaciens and L. kefiri co-cultured with various combinations of yeasts and auxiliary bacteria

*L. kefiri,* another ubiquitous species in milk kefir (Nejati et al. 2020), is applied in co-cultures development study here. *Ac. orientalis* is added to co-cultures, as it is the main acetate producer in kefir.

Cultures: *L. kefiranofaciens* K5 was grown in MRS medium for 5 days at 28 °C in anaerobic condition. *L. kefiri* 14 and *Ln. mesenteroides* Lgpr3 were grown in MRS medium and *Ac*. *orientalis* P2S2 in YPG, all for 24 h at 28 °C in aerobic condition. Yeasts S. *cerevisiae* DBVPG 10191, *Kz. turicensis* DBVPG 7206, *Kz. unispora* DBVPG 6429, *Kl. marxianus* DBVPG 6141, D. *anomala* DBVPG 10201 were grown in YPG medium at 28 °C, for overnight (about 18h, for S. *cerevisiae, Kz. unispora* and *Kl. marxianus)* or 2 days *(Kz. turicensis* and D. *anomala*).

Procedure: Milk fermentation was carried out in 96-well plate (Hydroplate HP96U; PreSens, Regensburg, Germany) at a temperature of 27 °C. Each well contain 260 µl milk (1.5% fat) inoculated with 3 % MRS-grown *L. kefiranofaciens* K5 alone, or in combination with 2% of MRS-grown *L. kefiri* 14. Co-cultures of *L. kefiranofaciens* and *L. kefiri* were further combined with various mixtures of yeasts (Y1-Y5)-P2S2-Lgpr3. Yeast species were mixed together, in a way that a lactose-hydrolyzing yeasts is mixed with two lactose-unhydrolyzing yeasts, shown in the Table 1. 1 % v/v of each yeast mixture was used in milk. *Ac. orinetalis* P2S2 and *Ln. mesenteroides* Lgpr3 each were inoculated of 0.4% v/v to milk in all mixed-cultures.

**Table 1**

| Yeast combination number | Accompanying yeasts | Lactose hydrolysing yeast |
|---|---|---|
| Y1 | *Kz. turicensis* DBVPG 7206, *Kz. unispora* DBVPG 6429 | *D. anomala* DBVPG 10201 |
| Y 2 | *S*. *cerevisiae* DBVPG 10191, *Kz. turicensis* DBVPG 7206 | |
| Y3 | *S*. *cerevisiae* DBVPG 10191, *Kz. unispora* DBVPG 6429 | |
| Y4 | *Kz. turicensis* DBVPG 7206, *Kz. unispora* DBVPG 6429 | *Kl. marxianus* BVPG 6141 |
| Y5 | *S*. *cerevisiae* DBVPG 10191, *Kz. turicensis* DBVPG 7206 | |

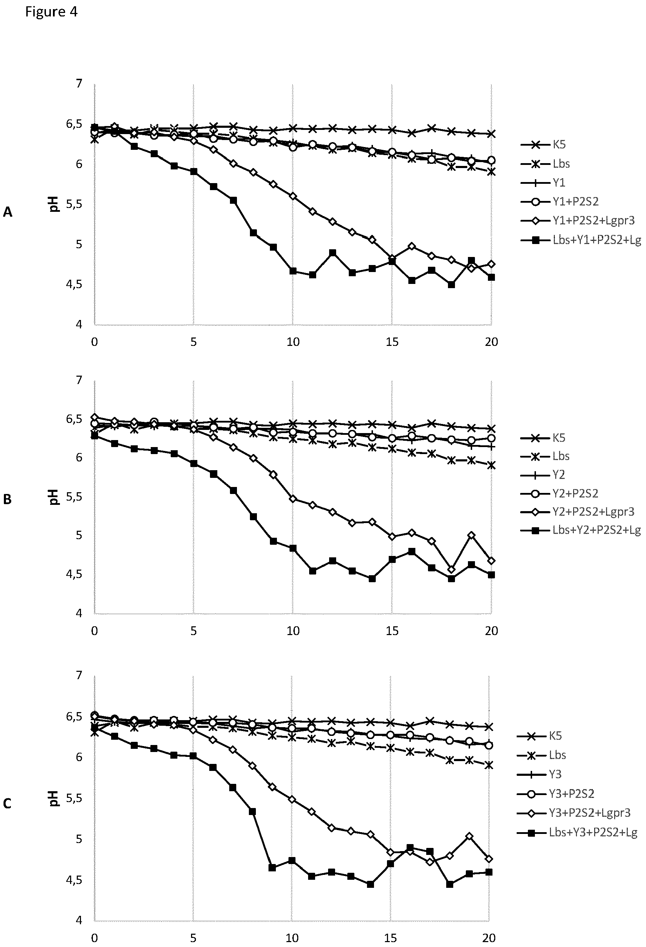

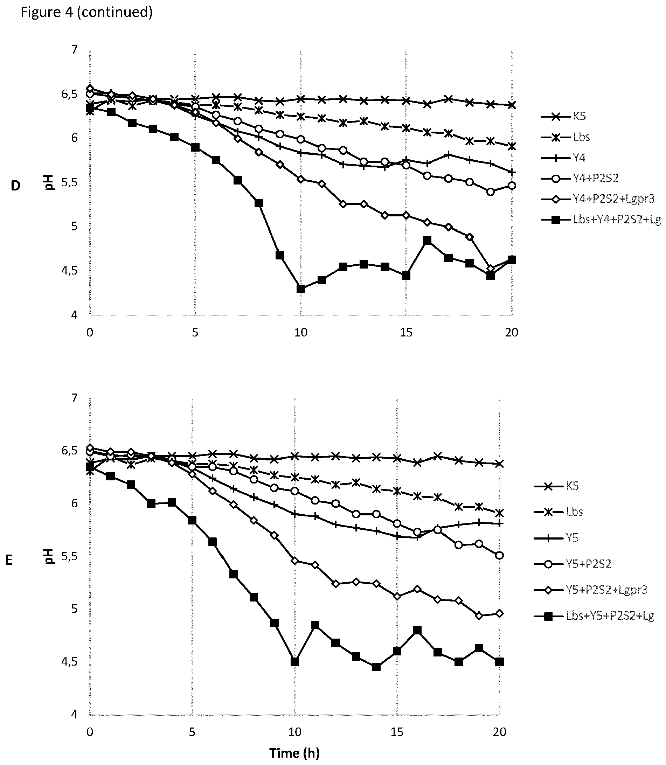

FIG 4 shows the course of pH of mixed of seven microorganisms starter cultures comprising of *L. kefiranofacies, L. kefiri, Ln. mesenteroides, Ac. orientalis* and three yeasts, as well as different controls were elaborated with lower than seven microorganisms to evaluate the interactions of different before-mentioned microorganisms during 20 h of milk fermentation.

As evident from the FIG 4, there is clear improvement in milk pH reduction inoculated by mixed-cultures comprising seven kefir-isolated microbial cultures, so that in less than 10 h, pH of ca. 4.7 was achieved. Milk inoculated with combinations of *Ln. mesenteroides, Ac. orientalis* and yeasts, without two Lactobacilli *L. kefiranofacies* and *L. kefiri,* were also able to reduce milk pH to below ca. 5.0 in around 15 h, which shows the synergistic effects among these species.

### Example 5. Milk acidification by combinations of various auxiliary species and strains

To analyze the capacity of auxiliary bacteria mixtures alone (without *L. kefiranofacies* and *L. kefiri)* to reduce the pH of the fermentation medium, the following experimentation was performed. Bacterial cultures *Ln. mesenteroides, Ac. orientalis* and *L. lactis,* named here as auxiliary microorganisms, are considered to be the main microorganisms responsible in milk pH reduction. However, milk acidification of each species is strain-specific and were analyzed in the following.

Cultures and procedure: Different combinations of above-mentioned species are tested. *Ln. mesenteroides* strains Lgpr3 and Lg2a (kefir origin), *Lc. lactis* strains NZ9000 and DC103 (non-kefir origin) and *Ac. orientalis* P2S2 (kefir origin), each cultivated for 24 h at 28 °C. The pellet of freshly-grown cultures were resuspended in PBS and inoculated to milk, in the way to achieve the final OD₆₀₀ 0.1 of each species/strain in milk equivalent to about 1*10⁶ to 5*10⁶. Milk fermentation was carried out in 96-well plate (Hydroplate HP96U; PreSens, Regensburg, Germany) at a temperature of 27 °C, each well contain 260 µl of inoculated milk (1.5% fat).

As is shown in FIG. 5 not only various combinations of *Ln. mesenteroides,* and *Lc. lactis* strains provide different ability in milk acidifications, so that strains *Lc. lactis* NZ9000 and *Ln. mesenteroides* LgPr3 showed stronger acidifying ability, compared to strains DC103 and Lg2a, in different combinations. According to these results, the combination *of Ln. mesenteroides* Lgpr3, *Ac. orientalis* P2S2 and *Lc. lactis* NZ9000 is used in the next example in mixed-cultivations with lactobacilli and yeasts species.

### Example 6. Effect of auxiliary bacteria concentrations in milk fermentation

To further assess the influence the propagation capability of co-cultures of *L. kefiranofaciens* and *L. kefiri* when cultured together with mixtures of yeast species and different auxiliary bacteria cell numbers of *L. kefiranofaciens* and *L. kefiri* were assessed species-specific using the method as described in Nejati et al. 2020 after cultivation of these microbial consortia in milk for up to 20 hours. Table 2 shows the results.

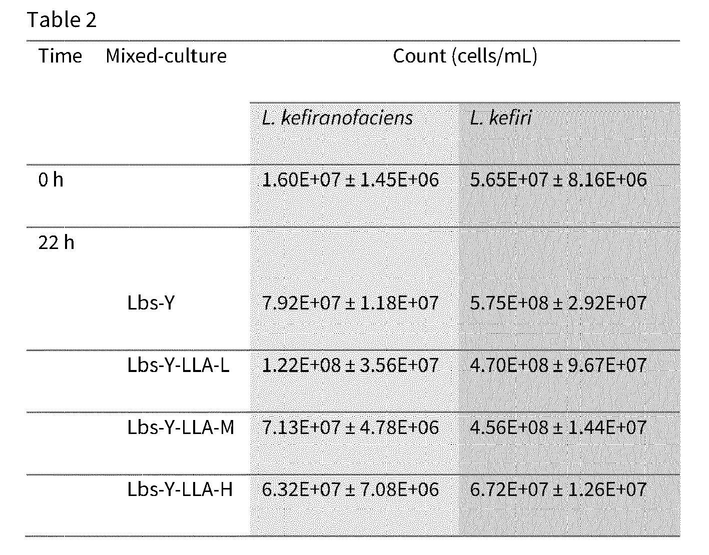

The aim here is to assess the impact of initial cell concentration of axillary bacteria LLA *(Ln. mesenteroides, Lc. lactis* and *Ac. orientalis; cells*/*mL fermentation medium)* on the course of pH as well as the counts of *L. kefiranofaciens* and *L. kefiri* in final fermented milk.

Cultures: *L. kefiranofaciens* K5 was grown in MRS medium for 5 days at 28 °C in anaerobic condition. *L. kefiri* 14, *Lc. lactis* NZ9000 and *Ln. mesenteroides* Lgpr3 were grown in MRS medium and *Ac. orientalis* P2S2 in YPG, all for 24 h at 28 °C in aerobic condition. Yeasts S. *cerevisiae* DBVPG 10191, *Kz. unispora* DBVPG 6429 and *Kl. marxianus* DBVPG 6141 were grown in YPG medium at 28 °C, for overnight (about 18 h).

Procedure: The pellet of all cultures was resuspended in PBS buffer, used for inoculation to milk. LLA cultures were inoculated to milk to achieve the individual initial concentrations of: A) 5.0E+04 ± 1.0E+04 cells/mL fermentation medium (LLA-L), B) 1.0E+05 ± 9.0E+04 cells/mL fermentation medium (LLA-M), and C) 5.0E+06 ± 1.0E+06 cells/mL fermentation medium (LLA-H). *L. kefiranofaciens,* starting cell concentration: 1.60E+07 ± 1.45E+06 cells/mL fermentation medium, *L. kefiri,* starting cell concentration: 5.65E+07 ± 8.16E+06 cells/mL fermentation medium. Cell numbers were measured via qPCR assay as described before. Milk fermentation was carried out in 96-well plate (Hydroplate HP96U; PreSens, Regensburg, Germany) at a temperature of 27 °C. Each well contain 260 µl milk (1.5% fat) inoculated with *L. kefiranofaciens, L. kefiri* and three yeasts (Y)(the three yeasts which were used in this example were named in section Cultures (previous paragraph)), further followed by inoculation of LLA cultures. After 22h of fermentation, the plate was centrifuged (3500 rpm, 15 min, RT) and the pellets were subjected to DNA extraction (NucleoSpin 96 DNA RapidLyze, Macherey-Nagel, Germany). Two microliter of purified DNA was used in total 20 µl qPCR assay for quantification of *L. kefiranofaciens* and *L. kefiri* according to the method as disclosed in (Nejati et al. 2020).

In FIG. 6, it is shown that the increasing of initial concentration of LLA cultures lead to faster acidification and reaching pH 4.7 in shorter time, 11 h when LLA-H was used compared to 20 h of LLA-M. The results of quantification of *L. kefiranofaciens* and *L. kefiri* shows that the highest *L. kefiranofaciens* cells are achieved in co-culturing with LLA-L, i.e. 1.22E+08 cells/mL for *L. kefiranofaciens* which is about 8 fold (7.63-fold) more cells after 22 h of fermentation and 5.75E±08 cells/mL for *L. kefiri,* which is about 10 fold (10.18-fold) more cells after 22 h of fermentation in here-mentioned mixed-culture. *L. kefiri* observed to be less sensitive to concentration of LLA cultures, although lower counts were detected by applying high concentration of LLA cultures. According to this example, the initial concentration of LLA cultures is the defining factor in acidification rate of milk in co-cultures comprising of *L. kefiranofaciens, L. kefiri* and yeasts, however in order to achieve to higher number of *L. kefiranofaciens* and *L. kefiri,* fast acidification is not preferable.

Similar results were obtained when co-cultures of *L. kefiranofaciens* and *L. kefiri* and optional yeasts were supplementing with the cell-containing supernatant of cultivations of auxiliary bacteria (e.g., both *Lactococcus lactis* and *Leuconostoc mesenteroides)* which were cultivated in cow's milk until they acidify the milk to pH 4.7 and below (data not shown).

### Example 7. Effect of dextran on milk acidification and proliferation of L. kefiranofaciens and L. kefiri in mixed-cultures

Exopolysaccharides, specially kefiran produced by *L. kefiranofaciens,* reported to have important role in traditional kefir in integrity of grains. The aim here it to evaluate the effect of dextran, an exopolysaccharide produced by *Ln. mesenteroides,* on acidification course of milk inoculated with mixed-cultures as well as *L. kefiranofaciens* and *L. kefiri* counts.

Cultures: *L. kefiranofaciens* ssp. *kefiranofaciens* K5 was grown in MRS medium for 5 days at 28 °C in anaerobic condition. *L. kefiri* 14, *Lc. lactis* NZ9000 and *Ln. mesenteroides* Lgpr3 were grown in MRS medium for 24 h at 28 °C in aerobic condition. Yeasts S. *cerevisiae* DBVPG 10191, *Kz. unispora* DBVPG 6429 and D. *anomala* DBVPG 10201 were grown in YPG medium at 28 °C, for overnight (except D. *anomala* for 2 days).

Procedure: The pellet of all cultures was resuspended in PBS buffer, used for inoculation to milk. *Lc. lactis* NZ9000 and *Ln. mesenteroides* LgPr3 cultures were inoculated to milk to achieve the initial concentrations of 1.0E+05-9.0E+05 cells/mL (measured via qPCR assay). Milk fermentation was carried out in a 24-well plate (Hydroplate HP96U; PreSens, Regensburg, Germany) at a temperature of 27 °C. Each well contain 1.8 mL milk (1.5% fat) inoculated with *L. kefiranofaciens, L. kefiri* and three yeasts, followed by inoculation of LL cultures. Dextran (from *Leuconostoc* spp. Mr ∼2000000) solution (3% w/v) was filter-sterilized and added to milk to achieve 0.1% w/v final concentration. Fermentation was continued for 35 h and at 22 and 35 h samples were taken for counting of *L. kefiranofaciens* and *L. kefiri* using the qPCR method as described by (Nejati et al. 2020). Lbs-Y: mixed-culture of *L. kefiranofaciens* and *L. kefiri* and yeasts, co-cultured with low, medium and high initial concentrations of bacteria *Ln. mesenteroides, Lc. lactis* and *Ac*. *orientalis* (LLA-L, LLA-M and LLA-H, respectively).

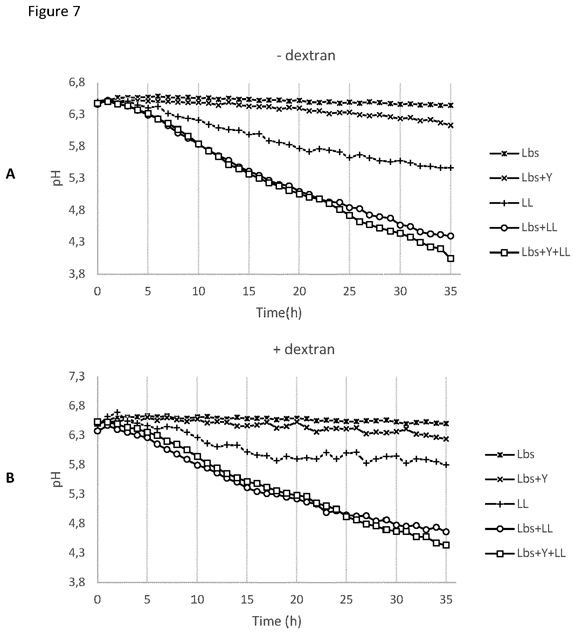

From FIG. 7 A-B, it is observed that the addition of dextran slightly delaying the acidification process, while the pH 4.7 was reached in milk without dextran and inoculated with complete mixed-culture *(L. kefiranofaciens, L. kefiri,* yeasts, *Lc. lactis* and *Ln. mesenteroides),* it was ca.4.9 in milk with dextran inoculated with the same microbial mixed-culture (FIG. 7).

Counting results for *L. kefiranofaciens* and *L. kefiri* shows that there is no difference in cell numbers in milk supplemented with or without dextran after 22 h, however, when prolonging fermentation time up to 35h following fermentation an dextran addition promisingly higher cell numbers (ca. two times more) was observed for both *L. kefiranofaciens* (1.59-fold increase) and *L. kefiri* (1.94-fold increase) in the presence of dextran in milk compared to the same approach without dextran (Dex) addition (cf. Table 3).

In essence these experiments show when grown in differently supplemented fermentation medium (cf. above) supplementations with yeast and additional with auxiliary bacteria and dextran will lead to the highest concentrations of these two core bacteria.

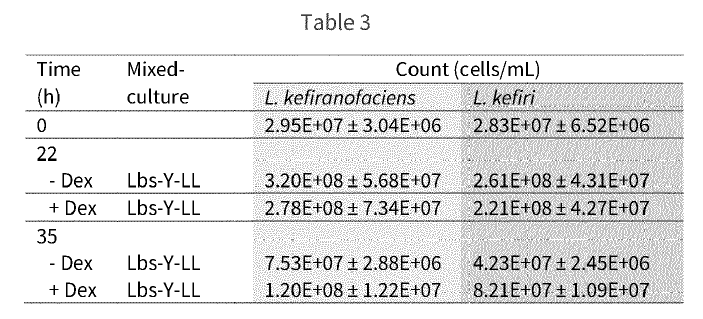

## Claims

1. A method to produce a synthetic kefir-like fermentation product by bacterial strain from a raw beverage source comprising:
a. providing a row beverage source and as fermentation microorganism at least one *Lactobacillus* selected from the group consisting of: an isolated *Lactobacillus kefiranofaciens* strain, an isolated *Lactobacillus kefiri* strain, a purified culture of *Lactobacillus kefiranofaciens,* a purified culture of *Lactobacillus kefiri* and mixtures thereof and optionally providing at least one yeast strain derived from natural kefirs;
b. adding the fermentation microorganism of step a. to a raw beverage source to receive a fermentation medium;
c. applying conditions to facilitate growth and division of the fermentation microorganism to allow for fermentation of the fermentation medium;
d. receiving the synthetic kefir-like fermentation product.

2. Method according to claim 1, wherein the raw beverage source is supplemented with a pre-fermented acidified raw beverage source derived by microbial fermentation and/or by enzymatic treatment comprising free amino acids and/or free oligopeptides or wherein the conditions to facilitate growth and division of step c. comprise that the fermentation medium is supplemented with a pre-fermented acidified raw beverage source derived by microbial fermentation and/or by enzymatic treatment comprising free amino acids and/or free oligopeptides.

3. Method according to claim 1 or 2, wherein the cell numbers of the at least one *Lactobacillus* are continuously monitored and regulated.

4. Method according to any of the previous claims, wherein the conditions to facilitate growth and division of step c. comprise keeping the temperature in a range from 16 °C and 37 °C, 18°C and 30°C or 20°C to 28°C.

5. Method according to any of the previous claims, comprising:
further adding to the raw beverage source or to the fermentation medium at least one isolated auxiliary bacterial strain or purified culture thereof derived from natural kefirs selected from the group consisting of: *Lactobacillus spp., Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobactersyzygii, Bifidobacterium* spp., *Enterococcus faecium, Enterococcus faecalis,* or mixtures thereof.

6. Method according to any of the previous claims, wherein the at least one optional yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof.

7. Method according to any of the previous claims, wherein step b. or c. further comprises:
adding at least one scaffold material to the raw beverage source or to the fermentation medium, wherein the at least one scaffold material is an exopolysaccharide selected from the group consisting of: kefiran, dextran, alternan, galactan, fructan, mutan, levan, reuteran, pullulan, curdlan and xanthan;
or is a natural polymer selected from the group consisting of: collagen and chitosan or is a synthetic polymer selected from the group consisting of: poly (ε- caprolactone) (PCL), poly (lactic acid) (PLA), poly (glycolic acid) (PGA), poly (3 hydroxybutyrate-co-3-hydroxyvalerate) (PHBV), and poly (lactic-co-glycolic acid) (PLGA).

8. Method according to any of the previous claims, wherein the raw beverage source is derived from mammal's milk and selected from the group consisting of: cow milk, goat milk, sheep milk, horse milk, buffalo milk.

9. Method according to any of the previous claims, wherein the at least one *Lactobacillus of step a* is added at a starting cell concentration of at least 1* 10⁶ cells/mL to 1* 10⁹ cells/mL fermentation medium and wherein the at least one *Lactobacillus* is grown by applying conditions of step c. to an end cell concentration, which is at least 7.5-fold higher, at least 10-fold higher or at least 15-fold higher than the starting cell concentration preferably within under 24h or 48h of fermentation.

10. Method according to any of the previous claims, wherein the at least one isolated auxiliary bacterial strain or purified culture thereof is added at a starting cell concentration of 1* 10⁴cells/mL to 1* 10⁸ cells/mL fermentation medium and wherein the at least one auxiliary bacterial strain is grown by applying conditions of step c. to an end cell concentration, which is at least 10-fold up to 1000-fold higher than the starting cell concentration preferably within under 24h or 48h of fermentation.

11. Method according to claim 9 or 10, wherein the end cell concentration in the kefir-like fermentation product is reached within 15h, 18h, 20h, 22h, 23h, 30h, 35h or 47h after adding the fermentation microorganism to the fermentation medium.

12. Microbial composition to produce a synthetic kefir-like fermented product comprising as fermentation microorganism:
a. at least one *Lactobacillus* of the group consisting of an isolated *Lactobacillus kefiranofaciens* strain, an isolated *Lactobacillus kefiri* strain, a purified culture of *Lactobacillus kefiranofaciens,* a purified culture of *Lactobacillus kefiri* and mixtures thereof; and
b. optional at least one yeast strain, wherein the at least one yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof.

13. Microbial composition according to claim 12, wherein the fermentation microorganism further comprise at least one auxiliary bacterial strain or purified culture thereof derived from natural kefirs selected from the group consisting of: *Lactobacillus spp., Lentilactobacillus* spp., *Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobacter syzygii, Bifidobacterium* spp., *Enterococcus faecium, Enterococcus faecalis,* or mixtures thereof.

14. Synthetic kefir-like fermented product comprising as fermentation microorganism:
a. at least one *Lactobacillus* of the group comprising an isolated *Lactobacillus kefiranofaciens* strain, an isolated *Lactobacillus kefiri* strain, a purified culture of *Lactobacillus kefiranofaciens,* a purified culture of *Lactobacillus kefiri* and mixtures thereof; and
b. optionally at least one yeast strain,
wherein the at least one yeast strain is an isolated strain or purified culture thereof derived from natural kefirs selected from the group consisting of: *Kluyveromyces marxianus, Saccharomyces cerevisiae, Kazachstania unispora, Kazachstania turicensis, Kazachstania exigua, Dekkera anomala, Debaryomyces hansenii, Saccharomyces boulardii* or mixtures thereof.

15. Synthetic kefir-like fermented product according to claim 14, wherein the fermentation microorganism further comprise at least one auxiliary bacterial strain or purified culture thereof derived from natural kefirs selected from the group consisting of: *Lactobacillus spp., Lentilactobacillus* spp., *Lactococcus lactis* ssp., *Leuconostoc mesenteroides* ssp., *Lacticaseibacillus* spp., *Limosilactobacillus* spp., *Lactiplantibacillus* spp., *Lactobacillus helveticus, Lactobacillus acidophilus, Lactobacillus delbrueckii, Lactobacillus delbrueckii* ssp., *Lentilactobacillus parakefiri, Lentilactobacillus buchneri, Lentilactobacillus parabuchneri, Lacticaseibacillus casei, Lacticaseibacillus paracasei, Lacticaseibacillus rhamnosus, Lactiplantibacillus plantarum, Lactiplantibacillus paraplantarum, Limosilactobacillus reuteri, Limosilactobacillus fermentum, Levilactobacillus brevis, Ligilactobacillus salivarius, Ligilactobacillus salivarius* ssp., *Acetobacter* spp., *Acetobacter orientalis, Acetobacter fabarum, Acetobacter okinawensis, Acetobacter lovaniensis, Acetobactersyzygii, Bifidobacterium* spp., *Enterococcus faecium, Enterococcus faecalis,* or mixtures thereof.

16. Synthetic kefir-like fermented product according to claim 14 or 15, wherein the kefir-like fermented product is a kefir-like fermented mammal's milk product selected from the group consisting of: cow milk, goat milk, sheep milk, horse milk, buffalo milk.

17. Synthetic kefir -like fermented product according to any of claim 14 to 16, wherein the at least one *Lactobacillus* has a concentration of at least 1* 10⁸ cells/mL kefir-like fermented product and/or the amount of produced kefiran is at least 0.5 g/L, at least 1.5 g/L, more preferred at least 2.0 g/L or at least 2.3 g/L most preferred at least 2.5 g/L kefir-like fermented product.
